(19) Europäisches Patentamt
European Patent Office
Office européen des brevets

(11) Publication number: **0 024 868**
**B1**

(12) **EUROPEAN PATENT SPECIFICATION**

(45) Date of publication of patent specification: **22.06.83**

(51) Int. Cl.³: **A 61 K 31/495**
//(A61K31/495, 31/47)

(21) Application number: **80302855.4**

(22) Date of filing: **19.08.80**

(54) **Schistosomicidal composition comprising oxamniquine and praziquantel.**

(30) Priority: **22.08.79 GB 7929246**

(43) Date of publication of application:
**11.03.81 Bulletin 81/10**

(45) Publication of the grant of the patent:
**22.06.83 Bulletin 83/25**

(84) Designated Contracting States:
**BE CH DE FR GB IT LI LU NL SE**

(56) References cited:
**DE - A - 2 312 134**
**GB - A - 1 166 538**
**GB - A - 1 441 554**

(73) Proprietor: **Pfizer Limited**
**Ramsgate Road**
**Sandwich Kent CT13 9NJ (GB)**
(84) **GB**

(73) Proprietor: **Pfizer Corporation**
**Calle 15 1/2 Avenida Santa Isabel**
**Colon (PA)**
(84) **BE CH DE FR IT LI LU NL SE**

(72) Inventor: **Brammer, Keith Whitby**
**Glebe House The Street Staple**
**Canterbury Kent (GB)**
Inventor: **Shaw, John Richardson**
**6 Short Street Chillenden**
**Canterbury Kent (GB)**

(74) Representative: **Wood, David John et al,**
**Pfizer Limited Ramsgate Road**
**Sandwich Kent CT13 9NJ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 024 868 B1

Schistosomicidal composition comprising oxamniquine and praziquantel

This invention relates to new pharmaceutical compositions having therapeutic properties in the treatment of schistosomiasis and is particularly concerned with the concomitant use of two schistosomicides already separately known to be useful in the treatment of schistosomiasis, but whose concomitant use for such treatment has not previously been proposed.

One of the schistosomicides is disclosed and claimed in GB—A—1,166,538 and is the compound generically known as oxamniquine, which is *dl*-6-hydroxymethyl-2-isopropylaminomethyl-7-nitro-1,2,3,4-tetrahydroquinoline. The other schistosomicide is disclosed and claimed in GB—A—1,441,554 and is the compound generically known as praziquantel, which is 2-cyclohexyl-carbonyl-4-oxo-1,2,3,6,7,11b-hexahydro-4H-pyrazino[2,1a]isoquinoline.

Thus according to the present invention there is provided a pharmaceutical composition for treating schistosomiasis comprising a compound of the formula:

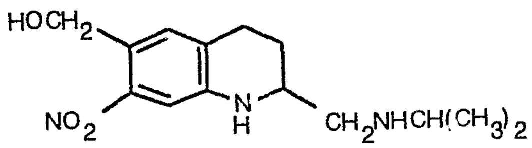

(oxamniquine)

or a pharmaceutically acceptable acid addition salt thereof; and a compound of the formula:

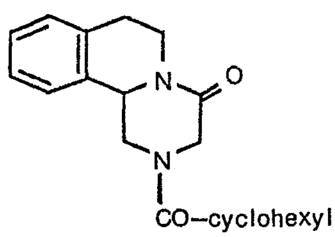

(praziquantel)

with or without a pharmaceutically acceptable diluent or carrier.

Pharmaceutically acceptable acid addition salts of oxamniquine are those which are formed with acids having pharmaceutically acceptable anions, e.g. the hydrochloride, hydrobromide, hydroiodide, sulphate or bisulphate, phosphate or acid phosphate, acetate, maleate, fumarate, lactate, tartrate, citrate, gluconate, saccharate and tosylate salts.

We have discovered that the concomitant use of oxamniquine and praziquantel is particularly and unexpectedly valuable in the treatment of schistosomiasis. Because of unexpected synergism, the concomitant use of these two compounds is more effective in the treatment of schistosomiasis than the use of either compound alone.

The compounds will be administered together in the form of a pharmaceutical composition comprising oxamniquine (or a pharmaceutically acceptable acid addition salt thereof) and praziquantel, the weight ratio (expressed as the free base of each compound) of oxamniquine to praziquantel preferably being from 1:0.5 to 1:5.0, and the composition will usually also comprise a pharmaceutically acceptable diluent or carrier. The compositions will preferably be in a form suitable for oral administration, e.g. in the form of tablets, capsules, emulsions, syrups or elixirs, but may be in a form suitable for parenteral administration, e.g. in the form of sterile injectable solutions. Suitable compositions can contain from 20—350 mg of oxamniquine and from 40—700 mg of praziquantel, the ratio of oxamniquine to praziquantel preferably being in the range from 1:0.5 to 1:5.0, most preferably 1:2.

Preferably the compounds are administered orally in a ratio of 1 part of oxamniquine to form 0.5—5.0 parts by weight of praziquantel. The most preferred weight ratio of oxamniquine to praziquantel is 1:2. The compounds are administered simultaneously, in tablets, capsules or syrups. The compounds can also be administered parenterally in similar ratios.

The daily dosage levels of each of the compounds will depend on the age and weight of the subject but (expressed as the free base of each compound) will generally be in the ranges of from 1—20 mg/kg of body weight of oxamniquine, and from 2.5—40 mg/kg of praziquantel, when administered orally or parenterally. The preferred dosage levels are 3—10 mg/kg of oxamniquine and 6—20 mg/kg of praziquantel. Thus for an average 70 kg subject, from 70—1400 mg (preferably 210—700 mg) of oxamniquine and from 175—2800 mg (preferably 420—1400 mg) of praziquantel would typically be administered per day in a single dose or up to 4 divided doses.

Compositions in forms suitable for oral or parenteral administration may be prepared by methods which are well known to those skilled in the art. When the compositions are in the form of tablets or capsules, these will generally contain excipients such as lactose or maize starch.

The concomitant administration of oxamniquine and praziquantel as described above is especially effective in the treatment of schistosomiasis due to the Puerto Rican strain of *Schistosoma mansoni.*

The following are Examples of the preparation of pharmaceutical compositions according to the invention:

Examples 1 to 3

The following ingredients are intimately mixed in the proportions given and then granulated by a conventional wet granulation technique. The resulting wet mass is dried and the dry mass screened in a conventional manner. The resulting granules are lubricated with a semi-synthetic triglyceride lubricant and filled into capsules to give a plug weight of 354 mg per capsule.

The proportions of the ingredients are given in parts by weight.

| Example | 1 | 2 | 3 |
|---|---|---|---|
| Oxamniquine (free base) | 160 | 80 | 40 |
| Praziquantel | 80 | 160 | 200 |
| Lactose | 70 | 70 | 70 |
| Maize Starch | 40 | 40 | 40 |
| Semi-synthetic triglyceride lubricant | 4 | 4 | 4 |
| | 354 | 354 | 354 |
| Wt. ratio oxamniquine: praziquantel | 1:0.5 | 1:2 | 1:5 |

Examples 4 to 6

The following ingredients are intimately mixed together in the proportions shown and then granulated by a conventional wet granulation technique. The resulting wet mass is dried and the dry mass is then screened in a conventional manner. The resulting granules are lubricated with a semi-synthetic triglyceride lubricant and the lubricated granules compressed into tablets of a suitable size (505 mg). The tablets are then coated with a film of hydroxypropylmethylcellulose by a conventional technique using conventional equipment to give tablets with a final coated weight of 525 mg.

The proportions of the ingredients are given in parts by weight.

| Example | 4 | 5 | 6 |
|---|---|---|---|
| Oxamniquine (free base) | 160 | 80 | 40 |
| Praziquantel | 80 | 160 | 200 |
| Lactose | 200 | 200 | 200 |
| Maize Starch | 60 | 60 | 60 |
| Semi-synthetic triglyceride lubricant | 5 | 5 | 5 |
| | 505 | 505 | 505 |
| Wt. ratio oxamniquine: praziquantel | 1:0.5 | 1:2 | 1:5 |

Examples 7 to 9

Suspensions suitable for oral administration are prepared with the following ingredients:

| Example | 7 | 8 | 9 |
|---|---|---|---|
| Oxamniquine (free base) | 50 g | 25 g | 12.5 g |
| Praziquantel | 25 g | 50 g | 62.5 g |
| Agar | 4 g | 4 g | 4 g |
| Sucrose | 550 g | 550 g | 550 g |
| Glycerin | 120 g | 120 g | 120 g |
| Sorbitol solution | 100 g | 100 g | 100 g |
| Sodium saccharin | 1 g | 1 g | 1 g |
| Sodium chloride | 10 g | 10 g | 10 g |
| "Polysorbate 80" (wetting agent) | 1 g | 1 g | 1 g |
| Flavourings | 2 g | 2 g | 2 g |
| Purified water | To 1 litre | To 1 litre | To 1 litre |
| Wt. ratio oxamniquine: praziquantel | 1:0.5 | 1:2 | 1:5 |

The suspensions are prepared by dissolving the "Polysorbate 80" (Registered Trade Mark) in purified water and adding praziquantel and oxamniquine, which are dispersed well. An agar solution is prepared by heating to boiling, followed by addition of the glycerin, sorbitol solution, sucrose, sodium saccharin and sodium chloride. The resulting solution is then cooled and added to the "Polysorbate 80"/praziquantel/oxamniquine solution, followed by the addition of the flavourings and making up to a 1 litre volume. The suspensions are then filled into bottles of a suitable size (100 ml), each 5 ml dose of which contains 375 mg of active ingredient.

The effectiveness of the method and compositions of the invention in the treatment of schistosomiasis has been assessed using a Puerto Rican derived strain of *Schistosoma mansoni* maintained by serial passage through Charles River (UK) CD—1 female albino mice and *Biomphalaria glabrata* snails. Mice are infected with 100 cercariae of *Schistosoma mansoni* and used 8—10 weeks post infection. The test compound(s) are suspended in Cremophor EL (Trade Mark of B.A.S.F., 10% in phosphate buffer pH 7, 0.2M). (When both oxamniquine and praziquantel are to be administered in combination, they are individually suspended in the Cremophor EL and the suspensions mixed together).

The resulting mixture is then administered to mice by oral gavage needle, 0.3 ml of suspension to a 30 gm mouse. 14 Days post treatment, the mice are killed by cervical dislocation and living worms recovered from the blood vessels by perfusion. The liver is removed and compressed between 2 glass plates and encapsulated male (i.e. dead) worms counted.

Effective doses (ED values) required to kill 50% and 99% of male worms are calculated from the regression line of the dose response data.

| Compound | $ED_{50}$ (mg/kg) | $ED_{99}$ (mg/kg) |
|---|---|---|
| Oxamniquine alone | 39 | 89 |
| Oxamniquine in combination with 25 mg/kg of praziquantel | 8.6 | 16.6 |
| Praziquantel alone | 156 | 296 |
| Praziquantel in combination with 12.5 mg/kg of oxamniquine | 4.9 | 27.2 |

Thus it will be seen that the total dose required for the combination to achieve equal efficacy to individual compound treatments at the $ED_{99}$ level is about half the dose using oxamniquine alone or

1/7th the dose using praziquantel alone. Also, a combination treatment using a 4—5 fold decrease in oxamniquine with an 8—10 fold decrease in praziquantel is as effective as individual treatments at the $ED_{99}$ level. The most effective combination in mice appears to be about 1 part by weight of oxamniquine with 2 parts by weight of praziquantel. These results indicate an unexpected synergistic effect between oxamniquine and praziquantel.

**Claims**

1. A pharmaceutical composition for use in treating schistosomiasis comprising a compound of the formula:

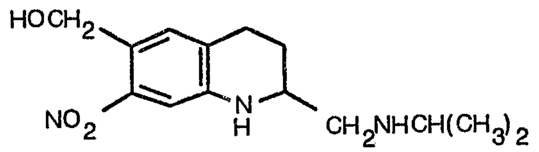

(oxamniquine)

or a pharmaceutically acceptable acid addition salt thereof; and a compound of the formula:

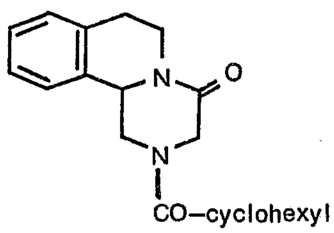

(praziquantel)

with or without a pharmaceutically acceptable diluent or carrier.

2. A composition as claimed in claim 1 wherein the weight ratio (expressed as the free base of each compound) of oxamniquine to praziquantel is from 1:0.5 to 1:5.0.

3. A composition as claimed in claim 2 wherein the ratio is about 1:2.

4. A composition as claimed in any one of the preceding claims which is in the form of a tablet, capsule, emulsion, syrup, elixir or sterile injectable solution.

**Patentansprüche**

1. Pharmazeutische Zusammensetzung zur Behandlung von Schistosomiasis enthaltend eine Verbindung der Formel

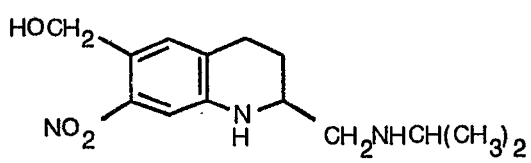

(Oxamniquin)

oder ein pharmazeutisch annehmbares Säureadditionssalz davon; und eine Verbindung der Formel

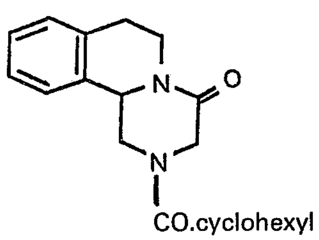

(Praziquantel)

mit oder ohne pharmazeutisch annehmbaren Verdünnungsmittel oder Träger.

2. Zusammensetzung gemäß Anspruch 1, worin das Gewichtsverhältnis (ausgedrückt als freie Base jeder Verbindung) von Oxamniquin zu Praziquantel 1:0,5 bis 1:5,0 beträgt.

3. Zusammensetzung gemäß Anspruch 2, worin das Verhältnis etwa 1:2 beträgt.

4. Zusammensetzung gemäß einem der vorgehenden Ansprüche in Form einer Tablette, Kapsel, Emulsion, Sirup, Elixier oder steriler injizierbarer Lösung.

**Revendications**

1. Composition pharmaceutique destinée au traitement de la schistosomiase comprenant un composé de formule

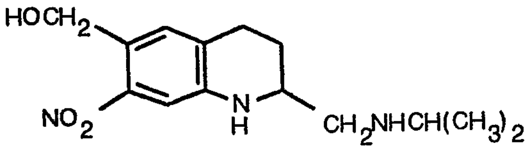

(oxamniquine)

ou un sel d'addition d'acide pharmaceutiquement acceptable de ce composé; et un composé de formule

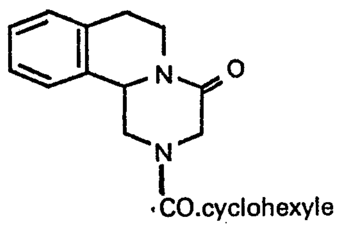

(praziquantel)

en présence ou en l'absence d'un diluant ou support pharmaceutiquement acceptable.

2. Composition suivant la revendication 1, dans laquelle le rapport en poids (exprimé en la base libre de chaque composé) de l'oxamniquine au praziquantel est de 1:0,5 à 1:5,0.

3. Composition suivant la revendication 2, dans laquelle le rapport est d'environ 1:2.

4. Composition suivant l'une quelconque des revendications précédentes, qui est sous la forme d'un comprimé, d'une capsule, d'une émulsion, d'un sirop, d'un élixir ou d'une solution injectable stérile.